# EUROPEAN PATENT APPLICATION

(11) **EP 1 130 104 A1**
(43) Date of publication of application: **05.09.2001**
(21) Application number: 00200535.3
(22) Date of filing: 16.02.2000
(51) Int. Cl.: C12N 15/82, A01H 5/00

(54) **Reduction of in planta degradation of recombinant plant products**

(71) Applicant: Stichting Dienst Landbouwkundig Onderzoek, 6708 PD Wageningen (NL)
(72) Inventor: Stevens, Lucas Henricus, 6708 JL Wageningen (NL); Jordi, Wilhelminus Jacobus Reiner Marie, 3511 ZC Utrecht (NL); Bosch, Hendrik Jan, 3572 ZM Utrecht (NL); Verhoeven, Henricus Adrianus, 4582 EN Schijndel (NL)
(74) Representative: Ottevangers, Sietse Ulbe

(57) **Abstract**

The invention relates to the field of the production and harvesting of plant products produced by biosynthesis in transgenic plants. The invention provides a method to increase the level of a desired product obtainable from a recombinant plant comprising allowing said plant to synthesise said product further comprising at least partly preventing degradation of said product.

## Description

The invention relates to the field of the production and harvesting of plant products produced by biosynthesis in recombinant plants, including plants having been provided with recombinant nucleic acid for example by infection or otherwise providing these with recombinant plant viral vectors or other suitable recombinant vectors.

Transgenic or recombinant plants are more and more used as cost efficient producers of useful proteinaceous substances such as recombinant biopharmaceutical proteins (antibodies, antigens, peptide and protein hormones, enzymes, and the like). The creation of recombinant proteins as e.g. medicaments or pharmaceutical compositions by pharmaco-molecular agriculture constitutes one of the principal attractions of transgenic plants; it is also the domain where their use is accepted best by the public opinion. In addition to the yield and the favourable cost which may be expected from the field production of recombinant proteins, transgenic plants present certain advantages over other production systems, such as bacteria, yeast and animal cells. They are for example devoid of virus which might be dangerous to humans, and can accumulate the proteins of interest in their "organs of storage", such as leaves, seeds or tubers. This facilitates their handling, their transportation and their storage at ambient temperature, and subsequent extraction or harvesting according to needs.

Other useful proteinaceous substances produced by recombinant plants are for example (heterologous) proteins (e.g. overproduced storage-proteins) rich in (essential) amino acids which are in general only present at low levels in a corresponding wild-type plant. Such protein enriched plants can provide a better and more balanced protein source for human and/or animal food.

Yet other useful proteinaceous substances produced by recombinant plants are for example (heterologous) enzymes or other metabolic proteins that are involved in a product's biosynthesis in that they help produce or are instrumental in producing plant metabolites. Examples of such (primary or secondary) metabolites are products such as proteins, carbohydrates, flavonoids, isoprenoids, terpenoids, fructanes, fatty acids, lipids, carotenoids, vitamines, alkaloids, phenolics, and other potentially useful plant products.

Although production of desired plant products (such as the above described proteinaceous substances and metabolites) in recombinant plants is now in principle feasible, there is much room for improvement. For example, it is desirable, considering the intricate and costly process of developing recombinant plants, to produce plants with as high production levels of the desired product as possible. Especially product levels at or around the time of harvest of the plant should be high, it is of no use to produce a recombinant plant that already produces the desired product at an earlier phase but wherein the product levels at harvest or in the harvested plant parts are comparatively low.

The invention provides a method to increase the level of a desired product obtainable from a plant or plant cell provided with a recombinant nucleic acid such as a recombinant plant or a plant having been provided with a vector, such as a viral vector, comprising a nucleic acid comprising allowing said plant or plant cell to synthesise said product further comprising providing for at least partial prevention of degradation of said product. For ease of reference, herein with plant not only a complete plant is meant, but also its constituting tissues or cells, such as plant cell cultures, algae cultures, recombinant plant cell cultures, and so on, which independently may be used as producer cultures of desired products. Such a desired recombinant plant product can itself of course be of a recombinant nature, e.g. a recombinant protein such as an antibody or an enzyme, but can be also a conventional product, such as a protein or any other primary or secondary metabolite that is produced by said plant via a metabolic process involving a recombinant enzyme, or be a product of which degradation in a catabolic process has been prevented via regulation through a recombinant protein.

The inventors have realised that it is not only biosynthesis per se that contributes to product levels, but that naturally occurring degradation processes of a once synthesised product contribute heavily to a decrease of said product as well as that naturally occurring degradation processes of a protein involved in said biosynthesis decrease said biosynthesis. By at least partly preventing degradation or degeneration of said product or protein involved in its biosynthesis, the balance is shifted, resulting in higher, and often more homogeneous, product levels at or around the time of harvest than when degradation was not prevented but let run its natural course.

In a preferred embodiment, the invention provides a method wherein said degradation is prevented by at least partly preventing catabolisation of said product. Catabolism, the normal degradation of complex products or molecules into smaller molecules, most often with release of reusable energy, is a normal process in plant development, whereby superfluous or little-used plant products are broken down for re-use and remobilisation of nutrients. Catabolic processes comprise for example proteolysis, break down of lipids, break down of carbohydrates or of primary or secondary metabolites, and so on, for example by specific enzymes such as proteases or lipases or by oxidative processes. Plants have an array of enzymes available for these catabolic processes, and the invention for example provides repressing at least one of these enzymes or catabolic processes to reduce, delay, or at least partly prevent degradation of the desired plant product, for example by coexpression of inhibitors of proteases or lipases, or by coexpression of antisense protease or lipase genes.

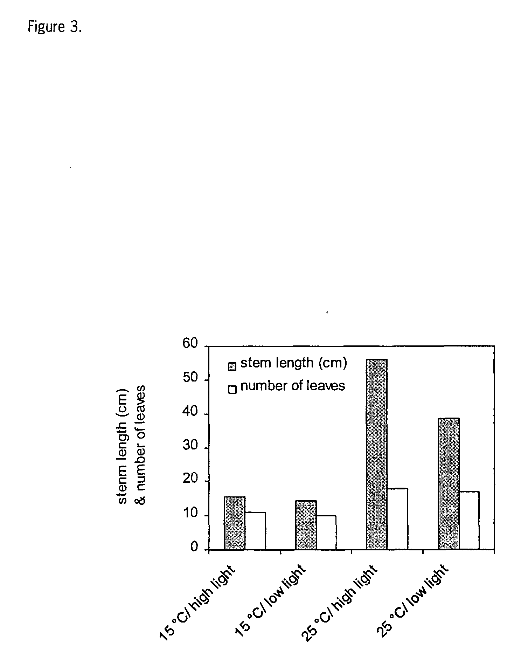

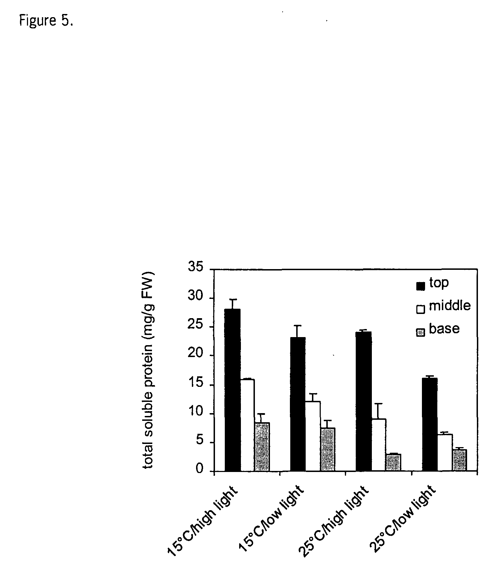

In a preferred embodiment however, the invention provides a method wherein said degradation is prevented by delaying senescence of said plant. Delaying senescence in plants in itself is already a known concept. Essentially, senescence functions as a recycling system of nutrients, which are translocated from the senescing tissue to young plant parts and reproductive organs. Therefore senescence normally occurs in correspondence with plant maturation and transition to the reproductive phase. This is perfectly illustrated by our observation presented in the detailed description that the plants that developed faster and started to flower, consistently exhibited lower protein levels than the plants that were less developed (fig. 3 and 5). To extend the growth of useful plants it is for example known to produce plants with delayed senescence (see for example Hensel et al., The Plant Cell 5:553-564, 1993; WO96/29858). However, the invention provides the insight to use or construct plants with delayed senescence in order to generate plants wherein degradation of a desired product is at least partly prevented. Herein it is for example provided to delay said senescence with or by the gene product of a senescence related nucleic acid with which said plant or an ancestor of said plant has been provided. A gene product herein is defined as RNA (be it sense or antisense) such as an mRNA encoding a protein related to or involved in senescence processes in said plant, or as a protein or functional fragment thereof, capable of influencing (preferably delaying, at least in specific plant parts) senescence.

Such a protein can for example be a regulator or catalyst in the biosynthesis of a plant growth regulator, such as a plant hormone (e.g. cytokinins, auxins, gibberellins, ethylene, abscisic acid or jasmonic acid), or a plant hormone receptor, or a functional fragment thereof. Preferably, said senescence is at least delayed in a harvestable part of said plant. Such harvestable parts are for example tubers, such as in potatoes, roots, such as in cassave or beets, leaves, such as in grass or tobacco, seeds, such as in cereals, e.g. corn, and soy. Specific expression of delayed senescence in such parts is also provided, such specific expression can for example be obtained using plant-tissue specific promotor sequences operably linked to a (senescence related) nucleic acid with which said plant or an ancestor of said plant has been provided. Alternatively, of course, it is provided to express or produce the desired product (mostly or only) in a specific tissue or tissues, and express the gene product related to the desired delayed senescence throughout the whole plant, or also tissue specific.

The invention also provides a recombinant plant capable of producing a desired product comprising a gene product allowing at least partly preventing degradation of said desired product. Such a plant can for example be obtained by crossing two recombinant plants, one having been provided with a recombinant nucleic acid related to or even encoding the desired product, another having been provided with a recombinant nucleic acid related to (at least partly) preventing degradation of products in said plant. Such a degradation-related nucleic acid comprise for example a nucleic acid encoding a gene product such as an antisense RNA but preferably comprises a senescence-related nucleic acid encoding a senescence-related protein or functional fragment thereof, such as a regulator or catalyst, or functional fragment thereof, of the biosynthesis of plant hormones, for example as shown herein in the accompanying examples.

Other ways that are provided to obtain a recombinant plant capable of producing a desired product comprising a gene product allowing at least partly preventing degradation of said desired product comprise further transformation of an already transformed or recombinant plant, to provide it with the desired two characteristics. Also, it is feasible to obtain such plants by infecting them with a recombinant vector, such as a recombinant viral vector (herein conveniently called virus) capable of expressing either or both of the desired nucleic acids, i.e. the delayed degradation-related nucleic acid can be viral-expressed in a plant already having been provided with a nucleic acid related to the desired product, or vice-versa, or both nucleic acids are viral-expressed in a (possibly further conventional) plant. The invention thus provides a method produce products of interest by infecting plants or plant cells with recombinant plant viruses which carry a gene or genes that incite the synthesis of the desired products. Such a gene can directly encode the protein of interest but can also encode proteins (enzymes) that induce the synthesis of other desired compounds.

Yet other ways comprise obtaining a plant or plant cell according to the invention by means of crossing a first (recombinant) plant for example comprising or having been provided with a delayed-senescence characteristic with a second plant having been selected for its already high production levels of a desired plant product. Such a second plant can be of the conventional type, having been selected for its high productivity by classical breeding techniques itself, or can be a recombinant plant, having been provided with high productivity by recombinant means.

In a preferred embodiment, the invention provides a plant wherein said desired product comprises a proteinaceous substance, such as an antigen, an antibody, a storage protein, a hormone, an enzyme, (or functional fragments thereof) and so on.

In yet another embodiment, the invention provides a plant wherein said desired product comprises a metabolite such as an isoprenoid, or another (primary or secondary) metabolite known in the art.

The invention also provides use, for example in crossing or breeding programmes, of a recombinant plant having been provided with a recombinant nucleic acid related to delayed senescence, such as a plant having been provided with leaf-specific cytokinine expression, in obtaining a plant according to the invention, as also explained in the detailed description and the examples herein.

The invention also provides a method for obtaining a desired plant product comprising cultivating a plant according to the invention to a harvestable stage and harvesting said plant or parts thereof, said method for example further comprising extracting said desired product from said plant, and the invention provides a desired plant product obtained or obtainable by a method according to the invention. The invention is further explained in the detailed description without limiting the invention.

### Detailed description

### Example 1: Production and catabolism of antibodies in tobacco.

Crop plants are considered as a potential system for the production of antibodies in bulk amounts at relatively low costs. Since the initial demonstration that transgenic tobacco is able to produce functional IgG1 from mouse (Hiatt et al., 1989), full-length antibodies, hybrid antibodies and antibody fragments like Fab and single-chain variable fragments (scFv) have been expressed in higher plants for a number of purposes. The produced antibodies can serve in health care and medicinal applications, either directly by using the plant as food ingredient, or as pharmaceutical or diagnostic reagent after purification from the plant material. In addition, antibodies may improve plant performance, e.g. by controlling plant disease, or by modifying regulatory and metabolic pathways (for reviews see Conrad and Fiedler, 1994; Ma and Hein, 1995; Smith, 1996; Whitelam and Cockburn, 1996).

IgG consists of two identical "heavy" (H) and two identical "light" (L) chains, which are folded in discrete domains that are stabilised by intermolecular disulphide bonds. The four chains are covalently linked by intramolecular disulphide bonds. It has been shown that for a proper assembly of the antibodies in plant cells it is essential that the proteins are targeted to the endoplasmic reticulum (ER), as in mammalian systems (Hein et al, 1991). This requires the presence of a signal sequence fused to the genes encoding the mature H and L chains. The origin of the required signal sequence is not critical, since sequences from plant, mouse and yeast have been successfully employed (Ma and Hein, 1995). Proteins that are co-translationally inserted into the ER are folded in a specific conformation before they can undergo further downstream transport, glycosylation and processing (Pagny et al, 1999). Generally, IgGl contains one, highly conserved glycosylation site in the Fc-region. Mouse IgGl produced by transgenic tobacco has been reported to be N-glycosylated with plant-specific glycan structures (Cabanes-Macheteau et al, 1999). The glycans attached to antibodies may play a role in structure stability, protection against proteolytic degradation and recognition by receptors (Dwek, 1996; O'Connor and Imperiali, 1996). The secretory system in principle releases the proteins into the extracellular space, the cell membrane, the vacuole or the tonoplast (Pagny et al, 1999). It has been experimentally confirmed that in plants the antibodies are excreted into the apoplastic space (Hein et al, 1991; van Engelen et al, 1994; De Wilde et al, 1998).

When plants are commercially used as heterologous system for large-scale production of functional antibodies high yields can be crucial. Yield is the net-result of synthesis and breakdown. So far, research has mainly been focused on obtaining balanced synthesis and proper assembly of the individual subunits, the latter being important for both functionality and stability of the antibody. Generally, relatively low yields of far below 1% are obtained. Little attention has been paid to proteolytic degradation *in planta* of the antibodies synthesised. The finding that along with the expression of full-length antibodies considerable amounts of Fab-like (De Neve et al, 1993) and F(ab')2-like (van Engelen et al, 1994) fragments are formed in transgenic tobacco indicates that degradation may play a significant role. When the protein is produced for pharmaceutical applications, its stability is even more important as a factor that determines product homogeneity. Massive proteolytic degradation occurs in particular during tissue senescence and during stress, when nutrients are remobilized for transport to other plant parts or when an increased capacity for synthesis of stress gene products is required. The induction of these processes can be triggered by a number of external (e.g. drought, temperature, mineral deficiency, shading, pathogen infection) and internal factors (e.g. growth regulators, reproduction, age) (for reviews see: Noodén, 1988; Smart, 1994; Buchanan-Wollaston, 1997). The developmental stage and the environmental conditions of the plant may therefore be important determinants for the proteolytic degradation of the antibodies synthesised.

The objective of the present study was to investigate whether proteolytic degradation *in planta* is a serious constraint for the production of antibodies by transgenic tobacco, and to study whether this could be resolved by preventing degradation. We therefore measured the levels of full-length monoclonal mouse IgG1 (MGR48) in tobacco (*Nicotiana tabacum* cv. Samsun NN) plants that were grown under four different climate conditions, and analysed the *in planta* proteolytic degradation of the antibody in plants with or without delayed senescence. This was done by establishing the profile of H chain content and the relative content of the major H chain breakdown product present in leaves of different developmental stages. In addition, the relative susceptibility of the antibody produced by the transgenic plants towards the proteolytic activity in tobacco leaf tissue was investigated. For this, the breakdown of MGR48 antibody purified from tobacco and of MGR48 antibody from mouse hybridoma cells was compared in the course of *in vitro* incubations with crude leaf extract from wild-type tobacco plants.

### Materials and methods

### Vector construction, tobacco transformation and selection of antibody producing line

The IgGl antibody was directed against subventral gland proteins of the nematode *Globodera rostochiensis*. The mouse hybridoma cell lines from which cDNAs of the MGR48 H and L chains were derived have been described by De Boer et al. (1996). The isolation of the cDNAs of H and L chains by means of PCR amplification, the vector construction; the construct encodes antigen binding antibody have been described elsewhere. Expression of the H chain is driven by the CaMV 35S promoter with duplicated enhancer (Kay et al., 1987), and the expression of the L chain by the TR2' promoter (van Engelen et al., 1994). Tobacco (*Nicotiana tabacum* cv Samsun NN) leaf discs were transformed essentially according to the method of Horsch et al. (1985). Stable transformed plants were maintained under sterile conditions on MS (Murashige and Skoog, 1962) agar medium (Duchefa) containing 3% (w/v) sucrose and subsequently were transferred to soil in the greenhouse. From leaves of 33 independent greenhouse grown transgenic plants, protein extracts were prepared and antibody expression levels were estimated by SDS-PAGE followed by immunoblot analysis using sheep-anti-mouse antibodies as described by Van Engelen et al. (1994). Based on these data, extracts of seven plants were selected for ELISA analysis. Microtiter plates were coated overnight with 200 ng per well of *G.rostochiensis* homogenate proteins in 50 mM sodium carbonate, pH 9.6 at 4 °C. Wells were washed with 0.1% (v/v) Tween20 in phospate buffered saline pH 7.2 (PBS), blocked for 2 h with 5% (w/v) non-fat dry milk powder in PBS and washed twice with 0.1% (v/v) Tween20 in PBS. Serial dilutions of extracts of the seven transgenic lines were added to the wells and incubated for 2 h. Hybridoma produced MGR48 antibodies were used as a standard. After washing 3 times with 0.1% Tween20 in PBS, sheep-anti-mouse alkaline phosphatase was added in PBS with 1% (w/v) non-fat dry milk, and incubated for 1 h. Plates were washed 5 times with 0.1% (v/v) Tween20 in PBS before adding 150 *µ*l substrate buffer (0.75 mg.ml⁻¹ p-nitrophenylphosphate in 0.1 M Tris/HCl pH 9.8, 5mM MgCl₂) was added and the A₄₀₅ was measured. One line (Line 31), showing the highest expression of antigen binding MGR48 (0.3%) was selected for all further experiments.

### Plant growth conditions

The transgenic tobacco plants were propagated in tissue culture on MS medium (Murashige and Skoog, 1962) containing 2% (w/v) sucrose, at 20 °C and under light/dark cycles of 14 h continuous light (60 *µ*mol.m⁻².s⁻ ¹) per day. Plants of ca. 5 cm in length were allowed to adapt to climate room conditions for one week at 18 °C, under light/dark cycles of 14 h continuous low light per day and relative humidity gradually declining from 97% to 70%. The plants were then grown on potting compost in climate rooms, either under low or high temperature (15 and 25 °C), and low or high light conditions (75 and 275 *µ*mol.m⁻².s⁻¹ during one continuous light period of 16 h.d⁻¹), which resulted in four groups of 9 plants. Each group was subdivided in 3 subgroups of 3 plants which were analysed separately. The night temperatures were kept 3 °C lower than the day temperatures. Relative humidity was 70%. Of every plant 3 portions of leaves were harvested, namely the top, the middle and the basal leaves. The top leaves are defined as the youngest leaves of at least 5 cm in length; the basal leaves are the first leaf at the bottom of the plant of at least 15 cm in length together with the first one in succession; the middle leaves are defined as the three leaves in the middle between the top and the basal leaves. The rest of the leaves were not analysed with respect to IgG and protein content. Immediately after harvest the plant material was frozen in liquid nitrogen and stored at -70 °C.

### Total soluble protein extraction

Leaves were ground in a precooled mortar under liquid nitrogen. To 1 gram of powdered tissue was added 5 ml of ice-cold protein isolation buffer (60 mM Tris pH 8.0, containing 500 mM NaCl, 10 mM EDTA, 30 mM β-mercaptoethanol and 0.1 mM phenylmethanesulphonyl fluoride). This was thoroughly mixed and centrifuged (12,000g, 0 °C, 3 min). The supernatant was stored at -80 °C before further analysis. Control experiments showed that during the procedure no detectable breakdown of antibody occurred.

### Purification of IgG1 from tobacco

Freshly harvested tobacco leaves were frozen in liquid nitrogen. The frozen leaves were powdered in a stainless steel blender which was precooled with liquid nitrogen. To 200 g of powdered plant material was added 600 ml of 5 mM EDTA, 0.5 mM phenylmethanesulphonyl fluoride, 20 mM sodiumbisulfite and 10 g of polyvinylpolypyrrolidone in 150 mM sodiumphosphate pH 7.0. The mixture was thawed and subsequently clarified by centrifugation (10,000g, 10 min, 4°C). From this homogenate a protein precipitate was prepared by ammonium sulphate precipitation (20-60% ammoniumsulphate saturation). This was resuspended in 90 ml of 100 mM NaCl in 50 mM sodiumphosphate pH 7.0 and, after clarification by centrifugation (20 min, 10,000g, 4°C) applied on a HiTrap Protein G bioaffinity column (column volume 5 ml; Amersham Pharmacia Biotech) which was equilibrated with 50 mM sodiumphosphate pH 7.0. Non-binding protein was washed off with 10 column volumes of the same buffer. Bound protein was subsequently eluted with 0.1 M glycine pH 2.7 and immediately brought to neutral pH by mixing with 1 M Tris pH 9.0 (50 *µ*l.ml⁻¹ of eluate). By means of buffer exchange on Sephadex G25 (PD-10 columns; Amersham Pharmacia Biotech) this protein fraction was brought in 50 mM MES pH 6.0 and applied on a cation exchange column (Mono S HR 5/5; Amersham Pharmacia Biotech) which was equilibrated with the same buffer. Protein separation was performed with a linear 0-0.3 M NaCl gradient over 17.5 ml in 50 mM MES pH 6.0.

### Electrophoresis, immunoblotting and quantification of IgG1

SDS-PAGE was performed as described by Laemmli (1970) on minigels of 10% or 12% acrylamide, and 0.32% bisacrylamide. The samples were prepared by mixing the protein extracts with loading buffer (4:1 v/v) which contained either 0 or 30 mM β-mercaptoethanol, and subsequent heating on a boiling waterbath for 2 minutes. The loading buffer consisted of 8% (w/v) SDS, 40% (v/v) glycerol and 0.1% (w/v) bromophenol blue in 200 mM Tris pH 6.8. After separation the proteins were either stained in the gel with Coomassie Brilliant Blue (R250) or immediately Western blotted. Blotting was performed by electrophoretic transfer of the protein bands onto nitrocellulose membranes for 1 h at 50 V in 1 mM Tris and 10 % (v/v) ethanol in 10 mM 3-cyclohexyl-amino-1-propane sulfonic acid pH 11 at room temperature. The membranes were blocked for 2 h at room temperature with 2% (w/v) bovine serum albumin and 0.2% Tween20 in PBS. Xylose- and fucose containing N-glycans were detected by incubating the blots directly with anti-horseradish peroxidase antibodies (Rockland). Antibody (and fragments) were detected by incubating the blots with anti-mouse IgG antibodies either conjugated with alkaline phosphatase, or, for densitometric quantification, conjugated with horse radish peroxidase. The alkaline phospatase reaction was performed with 0.1 mM 4-nitro blue tetrazolium (prepared from 92 mM stocksolution in dimethylformamide) and 0.1 mM 5-bromo-4-chloro-3-indolyl-phosphate 4-toluidine in 100 mM Tris pH 9.5 containing 100 mM NaCl and 10 mM MgCl₂ until the bands of the positive controls were clearly visible. For densitometric quantification of the protein bands of the H chain and H-chain breakdown product the blots were incubated for 2 h at room temperature with polyclonal sheep-anti-mouse IgG antibodies conjugated with horse radish peroxidase in 1% (w/v) BSA, 0.2% (v/v) Tween20 and 2% (v/v) protein isolation buffer in PBS. The blots were washed 5 times with 0.2% Tween20 in PBS pH 7.2 and subsequently incubated with ECL Western blotting detection reagent (Amersham Pharmacia). Films were exposed to the blots (1 to 10 min) and subjected to densitometric analysis using Scion Image software (release Beta3B). A concentration range of polyclonal mouse IgG (Sigma) in crude protein extract of wild-type tobacco leaves was used as standard.

### In vitro study of proteolytic activity

Crude protease extracts were prepared from top, middle and base leaves of wild-type Samsun NN plants which were at the start of flowering. The leaf tissue was ground in a precooled mortar under liquid nitrogen. Three ml of ice-cold phospate/citrate buffer pH 6.0 (0.4 M Na₂HPO₄/0.2 M citric acid, 1:0.58 v/v) containing 30 mM β-mercaptoethanol was added per gram of tissue powder. The suspension was gently mixed by using a tube pestle and centrifuged (12,000*g*, 0 °C, 3 min). The supernatants served as crude protease preparation and were stored at-80 °C before use. To compare the proteolytic capacity of the three leaf tissues either 15 *µ*l of each leaf preparation, or 7 *µ*g of leaf protein was mixed with 3 *µ*g of purified MGR48 IgGl from tobacco in a total volume of 120 *µ*l of the phospate/citrate buffer (titrated to either pH 4.5 or pH 7.0 with 1 M citric acid and 1 M Na₂HPO₄, respectively), and incubated at 30 °C. At different time intervals samples were taken, immediately mixed with SDS-PAGE loading buffer (4:1 v/v) containing 30 mM β-mercaptoethanol, and subsequently heated on a boiling waterbath for two min. The samples were stored at -80 °C before analysis. The susceptibility of MGR48 IgG1 from the plant was compared with the susceptibility of MGR48 IgGl from mouse hybridoma cells by incubating these antibodies with a crude protease preparation from wild-type tobacco. The MGR48 IgGl from mouse hybridoma cells was a kind gift of dr. Arjen Schots (Department of Nematology, Wageningen University, The Netherlands). 7.5 *µ*g of pure antibody was mixed with 120 *µ*l of crude leaf extract in a total volume of 240 *µ* l of the phospate/citrate buffer titrated to pH 4.5 with 1 M citric acid, and put in a closed tube on a 30 °C waterbath. At different time intervals samples were taken, immediately mixed with SDS-PAGE loading buffer (4:1 v/v) containing 30 mM β-mercaptoethanol, and subsequently heated on a boiling waterbath for two min. The samples were stored at -80 °C before analysis. The samples were analysed by electrophoresis on 15% SDS-PAGE gels and subsequent Western blotting as described in the previous section. The lanes were loaded with a mixture of 4 *µ*l of sample, 4 *µ*l of loading buffer and 7 *µ*l of water. Development of the blots and subsequent densitometric quantification of the H chain were performed as described in the previous section.

### Protein determination

Protein concentrations were determined according to Bradford (1976) using the Coomassie plus protein assay reagent from Pierce (Rockford, IL, USA) with bovine serum albumin as standard protein.

### Results

### Expression and purification of the antibody

MGR48 monoclonal antibody is an IgG1 type immunoglobulin from mouse directed against subventral gland proteins of the nematode *Globodera rostochiensis*. It contains one glycosylation site, namely in the Fc-region of each H chain. The MGR48 cDNAs of H and L chains were fused with a slightly modified antibody signal sequence and cloned into a single T-DNA. The expression of the H chain gene was under regulatory control of a constitutive CaMV 35S promotor, and the expression of the L chain gene was under control of a constitutive TR2' promotor (van Engelen et al., 1994). The construct was introduced into tobacco (*N.* tabacum cv. Samsun NN) by *Agrobacterium tumefaciens*-mediated leaf-disc transformation. The expression of functional antibodies was tested by Western blotting (not shown) and binding to *G. rostochiensis* antigen by means of ELISA (not shown). Based on these data the line with highest expression of functional antibodies was selected, propagated *in vitro* and transferred to the greenhouse for further experiments.

Immunoblotting of crude leaf extract of the transgenic greenhouse plants after SDS-PAGE under reducing conditions resulted in two major bands that positively reacted with polyclonal sheep-anti-mouse IgG and which corresponded with the H and L chains of the MGR48 antibody of hybridoma cells. In addition, some faint positive bands were observed, all exhibiting higher mobility than the H chain. No positive reaction was found with control extracts from wild-type plants.

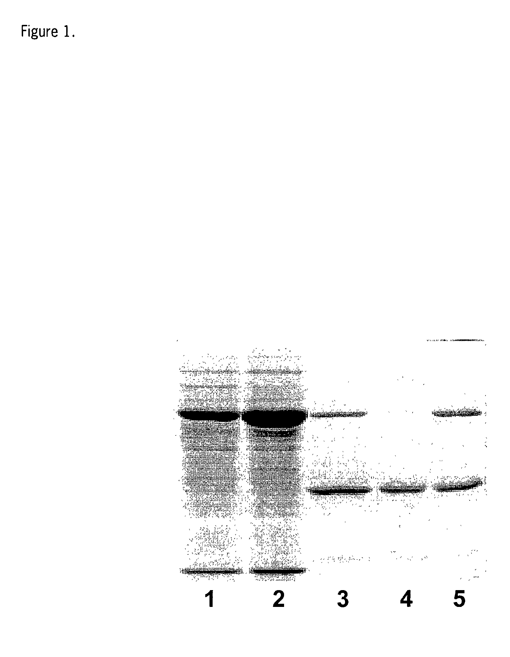

The antibody (and antibody fragments) were purified from crude leaf extract by ammoniumsulphate precipitation and subsequent Protein G-affinity chromatography. Comparison of immunoblots with Coomassie-stained PAGE gels indicated that all proteins present in the fraction that showed binding affinity to protein G ("total antibody") reacted with sheep-anti-mouse IgG. By means of cation-exchange chromatography the purified antibody could be separated into two fractions, one exhibiting weak binding (fraction I) and one exhibiting stronger binding (fraction II). The results of the successive steps in the purification procedure are depicted in figure 1, which shows the protein fractions on a SDS-PAGE gel run under reducing conditions. The fraction obtained after Protein G-bioaffinity chromatography mainly consisted of two proteins, a small one and a large one (fig.1, lane 3), the latter exhibiting a similar molecular mass as the large subunit of Rubisco (fig.1, lane 2). Interestingly, fraction I only exhibited the small band (fig.1, lane 4), whereas fraction II exhibited both small and large bands (fig.1, lane 5).

### Qualitative analysis of the plantibody

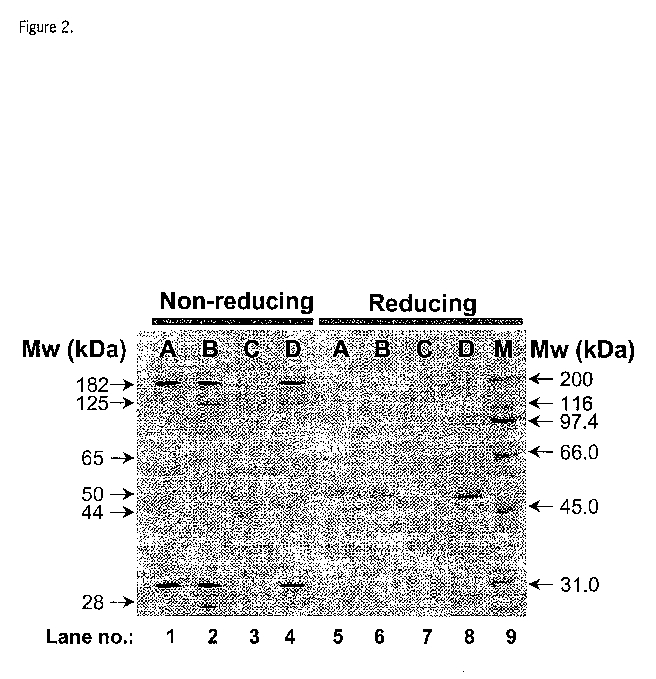

The purified total antibody (and antibody fragments) and fractions I and II were compared with MGR48 from mouse hybridoma cells by SDS-PAGE under reducing and non-reducing conditions (i.e. with and without β-mercaptoethanol). The small and large protein bands visible under reducing conditions showed identical mobility as the L and H chains of MGR48 from hybridoma cells, exhibiting molecular masses of 28 and 50 kDa (fig.2, lane 5, 6, 7 and 8), which is in fair agreement with the molecular masses calculated from the amino-acid sequences (26.8 and 51.3 kDa). By means of immunoblotting it was shown that the small monomer band of 28 kDa of both hybridoma and plant antibody reacted with antibody specifically directed against Fab fragments of mouse IgGl (results not shown). In addition, it was found that only the H chain of the plant antibody reacted with antibodies which specifically bind to plant-specific (xylose and fucose containing) N-glycans, whereas the H chain of the hybridoma antibody did not (results not shown). The fact that the small band of the plant antibody did not react with this glycan-specific antibody indicated that these monomer(s) did not contain the N-glycan part of the Fc-fragment. Under non-reducing conditions the antibody from MGR48 hybridoma cells showed only one band, representing the intact tetramer of two H and two L chains, with apparent total molecular mass of 182 kDa (fig.2, lane 1). The purified antibody from tobacco exhibited the same molecular mass, which confirmed the complete assembly of the tetramer in tobacco (fig.2, lane 2). In addition, one major band with apparent molecular mass of 125 kDa was found and three minor bands corresponding with 160, 65 and 44 kDa (fig.2, lane 2). The fractionation by cation-exchange chromatography had resulted in the separation of the small oligomer of 44 kDa (fraction I; fig.2, lane 3) and the complexes of 182, 160 and 125 kDa (fraction II; fig.2, lane 4).

These results showed that the transgenic tobacco plants produced intact MGR48 antibody which contained plant-specific N-glycans attached to the H chains. The presence of the disc-rete extra bands below 182 kDa on non-reducing PAGE gels strongly indicated that the produced antibody is broken down via some relatively stable intermediates. Most probably, the prominent protein band of 125 kDa represented a F(ab')2-like fragment, and the band of 44 kDa represented a Fab-like fragment, which implies that in tobacco the degradation of intact antibody starts with the proteolytic removal of (part of ) the Fc-region. This assumption is supported by the protein pattern on reducing PAGE gels which showed that the purified antibody mainly consisted of H chain (probably belonging to the intact antibody) and of protein with approximately the same molecular mass as the L chain (belonging to the intact antibody and to antibody fragments). Furthermore, the observation that the monomer(s) of 28 kDa derived from tobacco did not exhibit any fucose and xylose containing N-glycans indicated that the breakdown intermediates were devoid of the N-glycan part of the Fc-region.

### Effect of growth conditions and developmental stage on antibody levels

To find out whether climate conditions affect the net level of antibodies, the described transgenic tobacco plants were grown at low and high temperature (15 °C and 25 °C) under high and low irradiation (75 and 275 *µ*mol.m⁻² .s⁻¹ during one continuous light period of 16 h.d⁻¹), giving four groups of plants: (1) 15 °C/high light, (2) 15 °C/low light, (3) 25 °C/high light, and (4) 25 °C/low light. The plants were harvested and analysed after ca. 4 weeks, when the first plants started to flower.

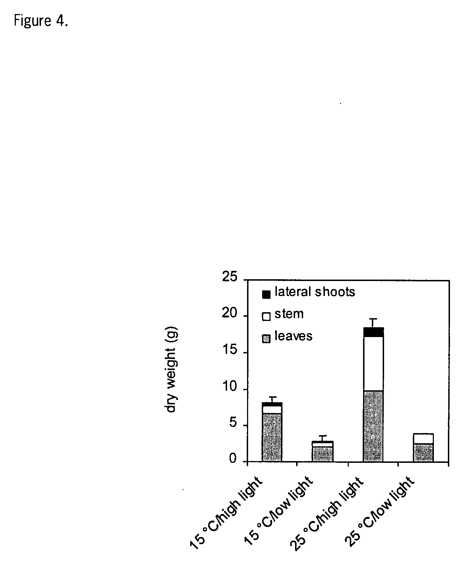

The treatments resulted in large differences with respect to biomass, plant length and number of leaves. Temperature strongly affected plant development. Plants grown at 25 °C developed faster than plants grown at 15 °C; the plants were taller and more leaves were produced (fig. 3). At the time of harvest the plants grown at 25 °C and high light had reached the stage of flowering whereas the plants grown at 15 °C only showed a nearly visible developing flower. The amount of applied light showed a positive correlation with biomass production (fig. 4); plants grown at 15 °C and high light showed even a higher production of dry weight then plants grown at 25 °C and low light (fig. 4).

To obtain an insight in the possible relationship between antibody content and developmental stage of the plant tissue, leaves of three different ages were analysed separately for the four groups of plants. These were young, growing leaves at the top of the plant (top leaves), mature, fully expanded leaves at the middle of the plant (middle leaves), and yellowing, old leaves at the plant bottom (base leaves).

Expressed on fresh weight basis, the top leaves contained more or less twice the amount of total soluble protein of the middle leaves, and the middle leaves in turn contained about twice the amount of the base leaves. This general profile of dramatically decreasing protein content from young to old leaves was observed for all four growth conditions tested (fig. 5). The plants grown at 25 °C contained less protein per amount of leaf tissue than the plants grown at 15 °C, in particular with respect to the base leaves. In fact this reflects the differences in rate of plant development at different temperatures that has been reported above. Also the amount of applied irradiation affected protein content. Leaf tissue contained more protein when grown under high light conditions. This effect was most pronounced for the plants grown at 25 °C.

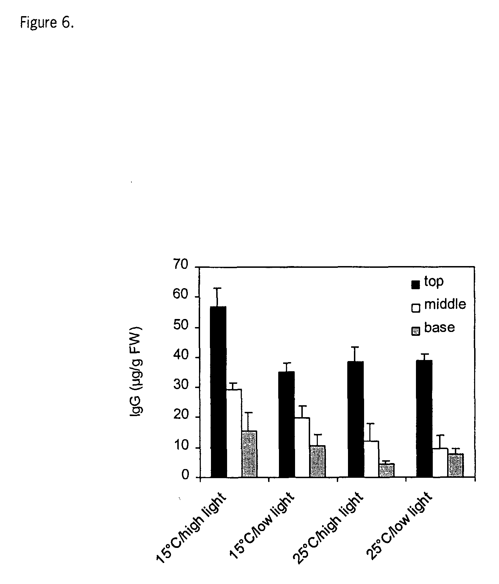

The amount of antibody present in the leaf tissue was determined by densitometric quantification of the H chain on immunoblots. Highest antibody levels were found in the top leaves (ca. 30 to 60 *µ*g.g⁻¹ of fresh weight; fig.6) and lowest levels were found in the base leaves (ca. 5 to 15 *µ*g.g⁻¹ of fresh weight; fig.6). However, since the profiles of IgG content (fig.6) essentially matched the profiles of protein content (fig.5), the amount of IgG expressed as percentage of total soluble protein in top (0.15-to 0.24%), middle (0.13 to 0.19 %) and base (0.14 to 0.21 %) leaves were rather similar. The plants grown at 25 °C contained less antibody per amount of leaf tissue than the plants grown at 15 °C. High light conditions favoured antibody content. In conclusion, highest levels of antibody per amount of fresh weight were found in the plants that were grown at 15 °C and high light.

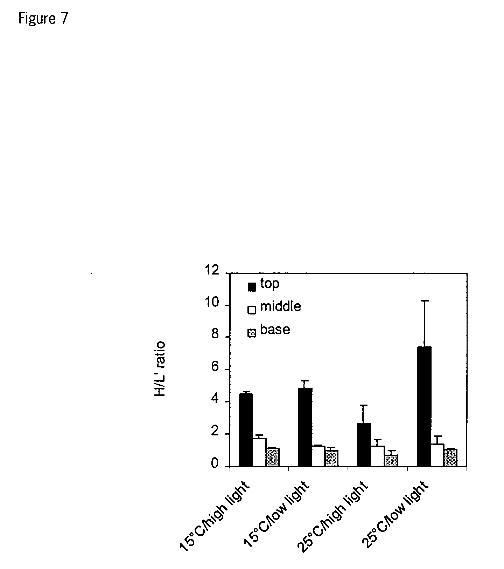

The qualitative analysis of the antibody and antibody fragments strongly indicated that the produced antibody is broken down via some relatively stable intermediates, of which the major H chain breakdown product co-migrated with the L chain on SDS-PAGE gels under reducing conditions. The ratio between the amount of H chain and the amount of putative H chain fragment that exhibited L-chain mobility ( H/L'-ratio) can thus be regarded as indicator for the proteolytic degradation of the antibody in the different leaf tissues. Therefore we determined also the relative amount of protein present in the bands of putative H chain fragment, using the same quantification procedure as applied for the detection of H chain. The affinity of the polyclonal sheep-anti-mouse antibody to the H chain and to the protein present in the bands of putative H chain fragment may differ; the H/L'-ratios presented here should therefore not be interpreted as molecular ratios. The results showed that the H/L'-ratios of the top leaves were 3 to 7 times higher than the H/L'-ratios of the middle and base leaves (fig.7). This large difference indicated that a substantial part of the H chain was broken down during the development of the leaf.

### In vitro degradation of plantibody MGR48 and mouse hybridoma MGR48

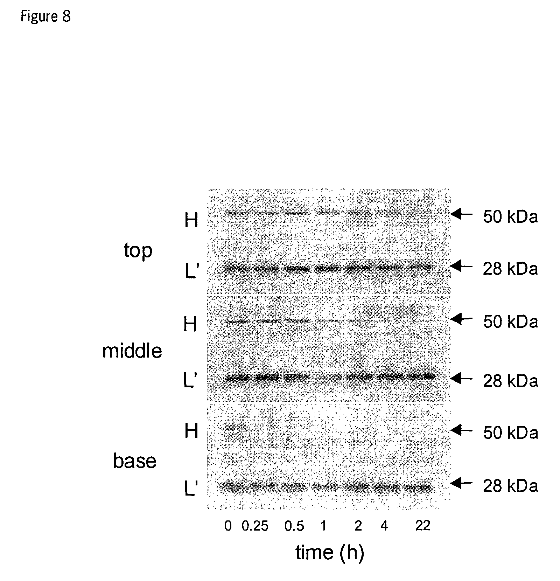

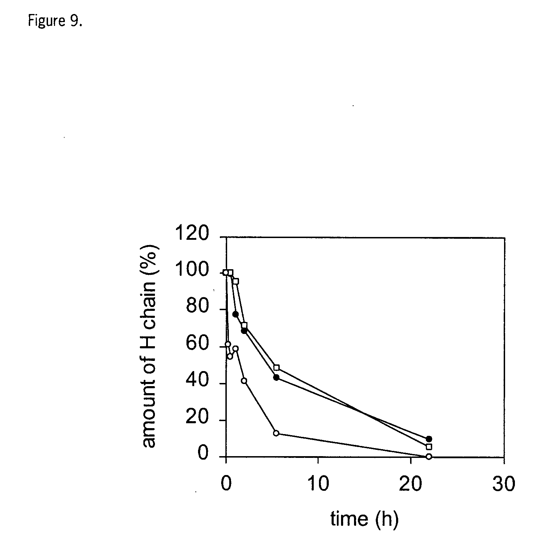

In order to corroborate the proteolytic potential of tobacco leaf tissue towards the plantibody we incubated crude protein extracts from top, middle and base wild-type tobacco leaves with antibody purified from the transgenic tobacco plants (fraction II in fig.1). The incubations were performed at pH 4.5 and pH 7. The reaction mixtures were analysed by immunoblotting after SDS-PAGE performed under reducing conditions. The band patterns of the immunoblots did show only little proteolytic degradation of the antibody at pH 7 (not shown). However, in the course of the incubations at pH 4.5 the H chain disappeared; the proteins that exhibited L chain mobility (i.e. the L chain and the putative H chain fragment) were relatively stable (fig.8). This degradation pattern *in vitro* was in complete agreement with the degradation observed *in planta* and therefore confirmed the conclusion that in tobacco the antibody is broken down by cleavage of (part of) the Fc region, resulting in a relatively stable intermediate that consists of subunits that exhibit the same electrophoretic mobility on SDS-PAGE gels as the L chain. Separate incubations with equal amounts of crude leaf extract instead of equal amounts of leaf protein showed that the proteolytic activity per amount of leaf tissue was significantly higher in the base leaves than in the top and middle leaves (fig.9). Although the middle leaves exhibited virtually the same proteolytic activity per amount of leaf tissue (fig.9) as the top leaves, they contained substantially less intact antibody (fig. 6). Presumably, breakdown increases with time of residence of the antibody in the leaf tissue.

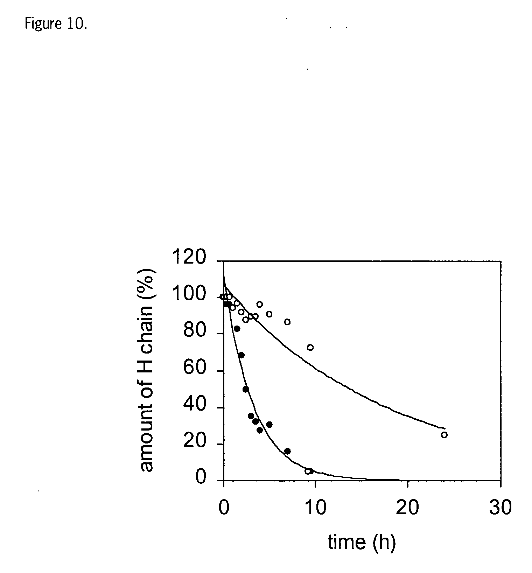

Antibodies are relatively stable proteins. The observed breakdown during leaf development prompted us to investigate whether the antibody produced by the plants is less stable than the antibody produced by the hybridoma cells. We therefore followed proteolytic degradation of equal amounts of hybridoma MGR48 and plantibody MGR48 in separate incubations with equal volumes of the same crude leaf extract from wild-type tobacco. The immunoblots showed that in the course of the incubations the H chain of MGR48 antibody produced by the tobacco plants disappeared with a higher rate than the H chain of MGR48 from hybridoma cells (fig. 10). Since the N-glycans attached to glycoproteins are assumed to play a role in folding, quaternary structure and stability of the protein (Dwek, 1996; O'Connor and Imperiali, 1996), the vulnerability of the plantibodies to proteolytic degradation may be due to the fact that they contain plant specific N-glycans instead of mouse N-glycans.

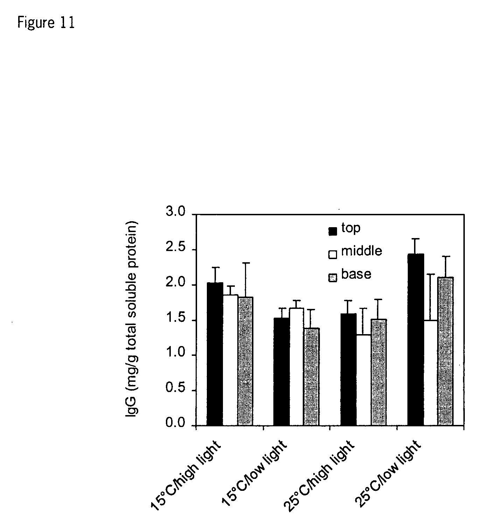

The results showed that the antibody content kept close pace with the level of total soluble protein, as is illustrated by the ratio between the relative amount of IgGl and total soluble protein, which was constant throughout the developmental stage of the leaf tissue (fig.11). This remarkable correlation makes it easy to predict changes of antibody levels from changes in amount of total protein. In theory however, there is no evident necessary link between both parameters. During senescence the changes in synthesis and breakdown are not the same for all proteins. Several enzyme activities are known to increase, in particular enzymes which are involved in nitrogen metabolism. Other proteins, such as chlorophyll-binding proteins, ribulose 5-phosphate kinase and Rubisco show a clear decrease (Noodén, 1988; Smart, 1994; Buchanan-Wollaston, 1997). Furthermore, the vast majority of soluble protein in green leaf tissue, being Rubisco, is localised in the chloroplasts, whereas after proper assembly and maturation in the ER and Golgi apparatus the antibodies are excreted into the apoplastic space (Hein et al, 1991; van Engelen et al, 1994; De Wilde et al, 1998). Therefore the antibodies probably are exposed to different pools of proteases.

Only few data are available about protease activity present in the apoplastic space. In developing oat leaves 16% of total acidic protease, active at pH 4.5 could be washed out from the intercellular space (van der Valk and van Loon, 1988). Significant endopeptidase activity was observed at acidic pH in the extracellular fluid from etiolated hypocotyls of *Phaseolus vulgaris* (Gomez et al, 1994). In the process of tracheary element differentiation of *Zinnea elegans* a serine protease, active at pH 5 is secreted during secondary cell wall formation (Groover and Jones, 1998). The acidic pH optima of these enzymes are consistent with the apoplastic pH, which may vary between pH 4 and pH 7, and for most plant species ranges between pH 5 and pH 6.5 (Grignon and Sentenac, 1991). The *in vitro* incubations of the antibody with crude enzyme preparations of wild-type tobacco at pH 4.5 and pH 7 indicated that the *in planta* degradation is catalysed by acidic proteases. This is in agreement with the assumption that the antibodies are degraded in the apoplast.

The band patterns obtained by SDS-PAGE and immunoblotting indicated that the antibody was degraded via some relatively stable intermediates, which probably were F(ab')2-like and Fab-like fragments. Similar results have been reported before (De Neve et al, 1993; van Engelen et al, 1994). This means that the proteolytic cleavage occurs between the Fab and Fc domains of the H chain, which is not unlikely, since this hinge region is susceptible for proteolytic cleavage by pepsin and papain.

One may only speculate about the possible causes of the difference in susceptibility to proteolytic degradation exhibited by the MGR48 antibody from the plant and the MGR48 antibody from the mouse. The finding that removal of saccharides has been shown to result in 60-fold increase in the rate of C_{H}2 cleavage by trypsin (Dwek et al. 1995) points at a role of the N-glycans. Plants and mammals differ in type of N-glycosylation. Plants have neither sialic acid nor Galß1-4 residues on their glycoproteins and exhibit carbohydrate motifs in their N-glycans that are not found in mammals (Lerouge et al., 1998). The glycan chains are N-linked on the inner face of the C_{H}2 domain and therefore are more or less buried inside the Fc region of the IgG molecule. From X-ray crystallography and NMR studies it is known that the nature of the sugar residues partly determines noncovalent binding interactions between the surface of the protein and the N-glycan chain; in particular Gal is associated with restricted motion of the N-glycans that fill the volume between the C_{H}2 domains (Dwek, 1996; O'Connor and Imperiali, 1996). The lack of a terminal Gal residue on the N-glycan, but also differences in absolute volume of the N-glycans may affect the conformation of the protein and consequently the accessibility to proteolytic cleavage.

The main conclusion of this study is that proteolytic degradation *in planta* can be a serious obstacle for the production of antibody in tobacco. It negatively affects yield as well as product homogeneity. The results strongly indicated that the major portion of the proteolytic degradation is part of the natural process of senescence, which starts when the plant tissue is mature and completely developed. Regulation of climate conditions does not offer a real solution to this problem. Temperature affected in particular the timing of antibody decline by controlling the rate of plant development. The final amount of antibody in mature leaf tissue could be slightly up-regulated by the application of high light conditions during growth; however, the antibody level per amount of total soluble protein was less sensitive to the amount of light, since total soluble protein content also increased with higher irradiance. Senescence-associated processes including protease expression also occur under stress conditions (Huffaker, 1990). This has implications for post-harvest handling and processing of the plant material. Furthermore, the presence of proteolytic activity in the source material may be a disadvantage for purification processes which make use of protein based bioaffinity techniques.

### Reduction of proteolitic breakdown of MGR48 antibody in plants with delayed senescence.

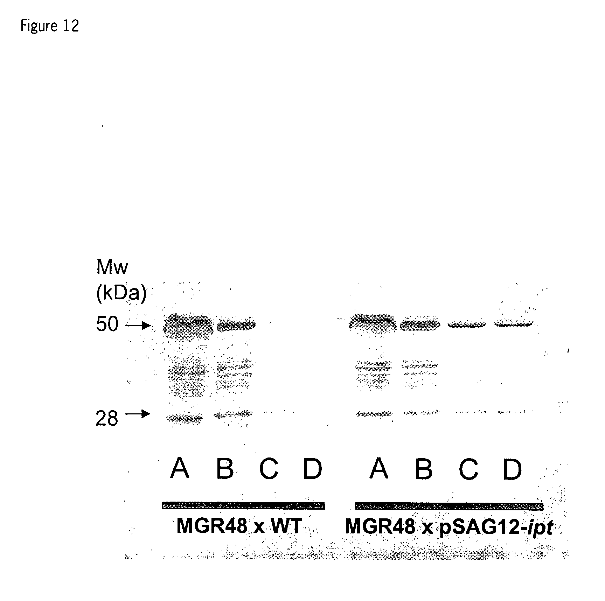

The effects of delay of senescence on the catabolism of a recombinant protein was investigated. Wisconsin tobacco plants exhibiting a genetic characteristic comprising delayed senescense (herein also called the StayGreen) phenotype (Gan & Amasino 1995) was crossed with the *Nicotianum tabacum* Samsun NN plant expressing the mouse monoclonal antibody MGR48. Flowers of StayGreen Wisconsin plants were pollinated with MGR48 plants. Of the progeny, plants were grown in the greenhouse and analysed for the expression of the antibody and the StayGreen phenotype. One plant displaying both traits was selected, propagated *in vitro* on MS medium (See M&M) and transferred back to the greenhouse on potting compost. As a control, MGR48 plants were crossed with the wild-type Wisconsin. StayGreen and control antibody expressing plants were grown under the same conditions, showed the same developmental characteristics and were harvested at the stage of flowering. The levels of MGR48 antibody in top, middle and two base leave levels of StayGreen and control plants were compared by means of immunoblotting (Fig. 12). The results demonstrated that higher levels of antibody were present in middle, and particularly in base leaves of StayGreen plants as compared to wild-type plants. This demonstrates that the StayGreen trait effectively protects the antibodies against catabolic breakdown.

### References to example 1

1. Bradford MM (1976) A rapid and sensitive method for the quantitation of microgram quantities of protein utilizing the principle of protein-dye binding. Anal Biochem 72: 248-254
2. Buchanan-Wollaston V (1997) The molecular biology of leaf senescence. J Exp Botany 48: 181-199
3. Cabanes-Macheteau M, Fitchette-Lainé A-C, Loutelier-Bourhis C, Lange C, Vine ND, Ma JKC, Lerouge P, Faye L (1999) N-Glycosylation of a mouse IgG expressed in transgenic tobacco plants. Glycobiology 9: 365-372
4. Conrad U, Fiedler U (1994) Expression of engineered antibodies in plant cells. Plant Mol Biol 26: 1023-1030
5. De Boer JM, Smant G, Goverse A, Davis EL, Overmars HA, Pomp H, Gent-Pelzer M van, Zilverentant JF, Stokkermans JPWG, Hussey RS, Gommers FJ, Bakker J, Schots A (1996) Secretory Granule Proteins from the subventral esophageal glands of the potato cyst nematode identified by monoclonal antibodies to a protein fraction from second-stage juveniles. Molecular Plant-Microbe Interactions 9: 39-46
6. De Neve M, De Loose M, Jacobs A, Van Houdt H, Kaluza B, Weidle U, Van Montagu M, Depicker A (1993) Assembly of an antibody and its derived antibody fragment in *Nicotiana* and *Arabidopsis.* Transgenic Research 2: 227-237
7. De Wilde C, De Rycke R, Beeckman T, De Neve M, Van Montagu M, Engler G, Depicker A (1998) Accumulation pattern of IgG antibodies and Fab fragments in transgenic *Arabidopsis thaliana* plants. Plant Cell Physiol 39: 639-646
8. Dwek RA, Lellouch AC, Wormald MR (1995) Glycobiology: 'The function of sugar in the IgG molecule'. J Anat 187: 279-292
9. Dwek RA (1996) Glycobiology: toward understanding the function of sugars. Chem Rev 96: 683-720
10.van Engelen FA, Schouten A, Molthoff, JW, Roosien J, Salinas J, Dirkse WG, Schots A, Bakker J, Gommers FJ, Jongsma MA, Bosch D, Stiekema WJ (1994) Coordinate expression of antibody subunit genes yields high levels of functional antibodies in roots of transgenic tobacco. Plant Mol Biol 26: 1701-1710
ll.Gomez LD, Casano LM, Rouby MB, Buckeridge MS, Trippi VS (1994) Proteolytic activity associated with the cell wall. AgriScientia 11: 3-11
12.Grignon C, Sentenac, H (1991) pH and ionic conditions in the apoplast. Annu Rev Plant Mol Biol 42: 103-128
13.Groover A, Jones AM (1998) Tracheary element differentiation uses a novel mechanism coordinating programmed cell death and secondary cell wall synthesis. Plant Physiol 119: 375-384
14.Hein MB, Tang Y, McLeod DA, Janda, KD, Hiatt A (1991) Evaluation of immunoglobulins from plant cells. Biotechnol Prog 7: 455-461
15.Hiatt A, Cafferkey R, Bowdish K (1989) Production of antibodies on transgenic plants. Nature 342: 76-78
16.Horsch RB, Fry JE, Hoffmann Nl, Eichholz D, Rogers SG, Fraley RT (1985) A simple and general method for transferring genes into plants. Science 227: 1229-1231
17.Huffaker RC (1990) Proteolytic activity during senescence of plants. New Phytol 116: 199-231
18.Kay R, Chan A, Daly M, McPherson J (1987) Duplication of CaMV 35 S promotor sequences creates a strong enhancer for plant genes. Science 236: 1299-1302
19.Laemmli UK (1970) Cleavage of structural proteins during assembly of the head of bacteriophage T4. Nature 227: 680-685
20.Lerouge P, Cabanes-Macheteau M, Rayon C, Fischette-Lainé A-C, Gomord V, Faye L (1998) N-glycoprotein biosynthesis in plants: recent developments and future trends. Plant Mol Biol 38: 31-48
21.Ma JK-C, Hein MB (1995) Plant antibodies for immunotherapy. Plant Physiol 109: 341-346
22.Murashige T, Skoog F (1962) A revised medium for rapid growth and bioassays with tobacco tissue cultures. Physiol Plant 15: 473-497
23.Nooden LD, Guiamét JJ, John I (1997) Senescence mechanisms. Physiol Plant 101: 746-753
24.O'Connor SEO, Imperiali B (1996) Modulation of protein structure and function by asparagine-linked glycosylation. Chemistry & Biology 3: 803-812
25.Pagny S, Lerouge P, Faye L, Gomord V (1999) Signals and mechanisms for protein retention in the endoplasmic reticulum. J Exp Botany 50: 157-164
26.Smart CM (1994) Gene expression during leaf senescence. New Phytol 126: 419-448
27.Smith MD (1996) Antibody production in plants. Biotechnolog Adv 14: 267-281
28.van der Valk HCPM, van Loon LC (1988) Subcellular localization of proteases in developing leaves of oats (*Avena sativa* L.). Plant Physiol 87: 536-541
29.Whitelam GC, Cockburn W (1996) Antibody expression in transgenic plants. TIPS 1: 268-272.

### Example 2: Production and catabolism of isoprenoids in transgenic petunia and tobacco

### Introduction

Isoprenoids are the largest class of natural compounds and are formed from C5 isoprene units. In most living organisms they play important roles, e.g. carotenoids involved in photosynthesis, mono- and sesquiterpenoids in cell to cell interactions or in interactions between organisms. Monoterpenes, the C10 branch of the isoprenoid family, were first investigated for their economically interesting value as flavor and fragrance additives in foods and cosmetics. Linalool is an acyclic monoterpene alcohol that has a peculiar creamy floral but no distinct sweet taste [Arctander, 1969]. In *Clarkia breweri* (GRAY) GREENE (Onagraceae) linalool, among other compounds, is responsible for the attraction of pollinating moths [Raguso, 1995]. The enzyme encoded by S-linalool synthase cDNA isolated from *Clarkia breweri* [Dudareva, 1996] and the plant purified enzyme has been shown to solely produce S-linalool [Pichersky, 1995]. The cDNA encodes for a cleavable peptide that can maximally be 8 aminoacids long [Dudareva, 1996], while other monoterpene and diterpene cyclases have cleavable transit peptides of 50-70 aminoacids [Bohlmann, 1998]. However, typical plastid targeting signal characteristics were found in the first 60 aminoacids of the cDNA [Cseke, 1998], indicating that linalool synthase is probably targeted to the plastids.

The downstream applications of genetically modified monoterpene metabolism are for example: a) Improving essential oil characteristics, b) altering floral scent in ornamentals, c) altering the flavor profile of a fruit or vegetable, d) altering the ecological environment of a plant, e) production of highly valuable monoterpenes and f) producing larger quantities of monoterpenes.

In our study we aimed to constitutively express the *Clarkia breweri* linalool synthase in *Petunia hybrida* W115 and *Nicotiana tabacum* under control of a constitutive double enhanced CAMV 35S promoter. In floral tissues [de Vos, 1999], as well as in other organs of *Petunia hybrida* W115, which is an easily transformable plant [Horsch, 1985], no monoterpenes could be detected by GC-MS studies, whereas in tobacco, linalool is observed only in the flowers during anthesis.

### Introduction of the linalool synthase gene (lis) in Petunia hybrida W115 and Nicotiana tabacum.

A *Clarkia breweri* pBluescript cDNA clone, Lis73 (kindly provided by Dr. E. Pichersky), coding for linalool synthase (Dudareva, 1996), was used for all experiments. The linalool synthase BamHI, SalI cDNA insert was ligated between the BamHI site of an enhanced CaMV-35S promoter and the SalI site before a Nos terminator sequence of a pFlapl0 vector using T4DNA ligase (GibcoBRL). The ligation product was transformed to *E.coli DH5a* competent cells, and transformed colonies were grown O/N at 37°C. The expression cassette was removed from the resulting vector by using PacI and AscI restriction enzymes (NEB, England) and ligated into the binary vector pBINPLUS after digestion with PacI and AscI. The resulting binary expression vector was transformed into *Agrobacterium tumefaciens* strain LBA4404. Colonies were checked after transformation by back-transformation to *E. coli DH5a* competent cells. Leaf cuttings of *Petunia hybrida* W115 and *Nicotiana tabacum* cv petit Havanna were transformed with *Agrobacterium tumefaciens* strain LBA4404 carrying the relevant binary plasmids and regenerated using a standard plant transformation protocol [Horsch, 1985]

21 transgenic petunia plants were obtained. All plants were phenotypically normal and showed a normal development as compared with non-transformed petunia plants. Total RNA was extracted from young leaves of two non-transformed control plants, from 10 plants transformed with an empty binary vector, and from 21 plants transformed with *lis73.* Northern blot analysis of linalool synthase mRNA expression in a subset of 5 transformed plants by indicated large differences in expression levels between the various independent transformants. On the basis of these results, two transgenic petunia plants were selected for further analysis; plant 17 with intermediate expression level and plant 24 with high *lis73* mRNA expression level in the leaves.

20 transgenic tobacco plants were obtained. All plants were phenotypically normal and showed a normal development as compared with non-transformed tobacco plants. All tobacco plants were directly tested for linalool expression (see below).

### Expression of linalool in Petunia hybrida W115 and Nicotiana tabacum

The presence of linalool was determined by GC-MS analysis of plant tissue samples. The tissues to be analyzed were collected in the greenhouse and frozen in liquid nitrogen. 200mg frozen material was homogenized and transferred to a mortar containing 1.5 ml 5M CaCl₂ and a small amount of purified sea sand. The material was rapidly and thoroughly ground with a pestle, inhibiting enzymatic reactions as much as possible. 0.75 ml of the material was introduced into a 1.8 ml GC vial containing a small magnetic stirrer. The vial was then closed with an aluminum cap with a PTFE/Butylrubber septum. Subsequently the vial was placed in a 50 °C waterbath and preheated for 20 minutes while stirring. The headspace sampled during 30 minutes with a 100*µ* PDMS Solid Phase MicroExtraction (SPME) fiber (Supelco, Belfonte PA USA).

Capillary gas chromatography- mass spectrometry (GC-MS) analysis was performed using a Fisons 8060 gas chromatograph directly coupled to a MD 800 mass spectrophotometer (Interscience, Breda, the Netherlands). A HP-5 column (50m x 0.32 mm, film thickness 1.05 *µ*m) was used with He (37 kPa) as carrier gas. GC oven temperature was programmed as follows: 2 min 80°C, ramp to 250°C at 8°C/ min and 5 min 250°C. Mass spectra in the electron impact mode were generated at 70 eV. The compounds were identified by comparison of GC retention indices and mass spectra with those of authentic reference compounds. Injection was performed by thermal desorption of the SPME fiber in the injector at 250°C during 1 min using the splitless injection mode with the split valve being opened after 60sec [Verhoeven, 1997]. Peaks were quantified using a calibration curve obtained by injection of known amounts of S-linalool. Chirality was checked by analysis on a chiral column (Dex-beta120, Supelco, Belfonte PA USA).

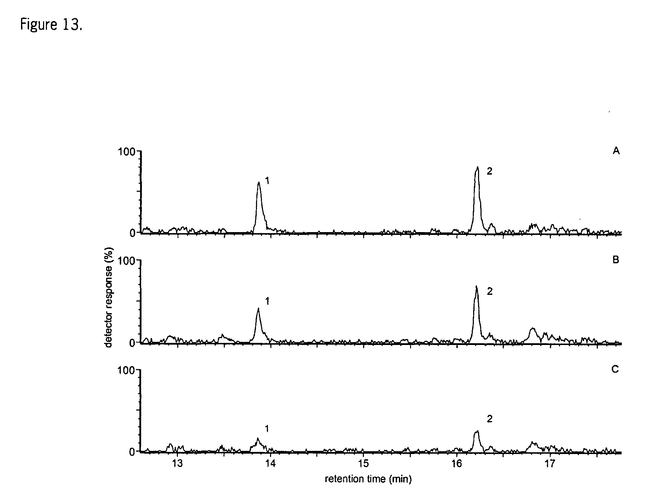

No linalool could be detected in any tissue of the control petunia plants. In general, linalool could be detected in leaves and other green parts of plants transformed with *lis73*. The level of linalool produced was determined by comparison with known amounts of linalool to be in the range of 100 ng/g fresh weight in leaves of plant line 17 (data not shown). The effect of age on the leaves was studied by analysis of three leaves of plant 24 (Fig 13), originating from the top (panel A), the middle (panel B) and base level (panel C). Peak 1 represents linalool and peak 2 represents α-terpineol, a chemical derivative of linalool. As demonstrated by the disappearance of peaks 1 and 2, both linalool and α-terpineol disappeared from older leaves.

All 20 transgenic tobacco plants carrying the *lis73* gene were transferred to the greenhouse, grown to maturity and subsequently analysed for linalool expression. Leaves were harvested, headspaced using SPME extraction, and analysed by GC/MS as described above. Linalool was identified by its retention index, mass spectrum and verified by injection of commercially available linalool (Fluka). Like in the transgenic petunia plants, expression of linalool in older leaves is significantly reduced as compared to expression in younger leaves (data not shown).

### Reduction of catabolism of isoprenoids in StayGreen plants

It was investigated if delay of senescence could also reduce catabolism of the isoprenoid linalool. Wisconsin tobacco plants carrying the pSAG12-ipt gene exhibiting the StayGreen phenotype (Gan & Amasino 1995) was crossed with the *Nicotianum tabacum* Petit Havanna SR1 which synthesizes linalool due to the linalool synthase transgene. Flowers of the StayGreen Wisconsin plants were pollinated with linalool plants. Of the progeny, plants were analysed for the expression of linalool and the StayGreen phenotype. One plant displaying both traits was selected for further analysis. As a control, linalool plants were crossed with wild-type Wisconsin. StayGreen and control linalool expressing plants were grown under the same conditions, showed the same developmental characteristics and were harvested at the stage of flowering. The levels of linalool in top, middle and base leave levels of StayGreen and control plants were compared by means of GC-MS analysis. The results demonstrated that higher levels of linalool were present in the older leaves of StayGreen plants as compared to wild-type plants. This demonstrates that the StayGreen trait also effectively protects a metabolite like linalool against catabolic breakdown. It also demonstrates that protection of catabolism is effective in different subcellular compartments which further demonstrates the versatility of this approach.

### References to example 2

1. Arctander S (1969) Perfume and flavour chemicals (aroma chemicals) In: S. Arctander ed, Ed first, Vol II: monographs no 1791 to 3102 (K through Z). Montclair (N.J.) pp 904
2. Bohlmann J, Meyer Gauen G, Croteau R (1998) Plant terpenoid synthases: Molecular biology and phylogenetic analysis. Proceedings of the National Academy of Science of the United States of America. April 95: 4126-4133
3. Cseke L, Dudareva N, Pichersky E (1998) Structure and evolution of linalool synthase. Mol. Biol. Evol. 15: 1491-1498
4. de Vos CHR, Verhoeven HA, Tunen AJv (1999) Flower volatile production in *Petunia hybrida.*
5. Dudareva N., Cseke L, Blanc VM, Pichersky E (1996) Evolution of floral scent in Clarkia: novel patterns of S-linalool synthase gene expression in the C. Breweri flower. Plant Cell 8: 1137-1148
6. Horsch, RB, Fry JE, Hoffmann NL, Eichholtz D, Rogers SG, Fraley RT (1985) A simple and general method for transferring genes into plants. Science, USA 227: 1229-1231
7. Pichersky E, Lewinsohn E, Croteau R (1995) Purification and characterization of S-linalool synthase, an enzyme involved in the production of floral scent in Clarkia breweri. Arch. Biochem. Biophys. 316: 803-807
8. Raguso RA, Pichersky E (1995) Floral volatiles from Clarkia breweri and c. concinna (Onagraceae): Recent evolution of floral scent and moth pollination. Plant Systematics and Evolution 194: 55-67
9. Winterhalter P, Katzenberger D, Schreier P (1986) 6,7-Epoxy-linalool and related oxygenated terpenoids from Carica papaya fruit. Phytochemistry 25: 1347-1350
10. Gan S, Amasino RM (1995) Inhibition of leaf senescence by autoregulated production of cytokinin. Science 270: 1986-1988
11. Verhoeven, H., Beuerle T, Schwab W (1997) Solid-phase microextraction: Artefact formation and its avoidance. chromatographia 46: 63-66

### Example 3

Plants can also be made to produce products of interest by infecting them with recombinant plant viruses which carry a gene or genes that incite the synthesis of the desired products. Such a gene can directly encode the protein of interest but can also encode proteins (enzymes) that induce the synthesis of other desired compounds.

Improved virus mediated expression of a peptide hormone in StayGreen plants

A single chain bFSH gene fusion (sc-bFSH) was constructed. By overlap PCR, the carboxyl end of the beta subunit was fused to the amino end of the alpha subunit (Sugahara *et al*., 1996). The sc-bFSH subunit was subcloned immediately downstream of an additional coat protein promoter in a TMV expression vector (Donson *et al*., 1991; Kumagai *et al*., 1993). *In vitro* transcripts were made and constructs were rubbed mechanically onto both wild type *Nicotiana* tabacum L. cv. Wisconsin plants and onto its StayGreen variant carrying the pSAG12-*ipt* gene. Crude protein extracts of infected plants were used for Western blot analysis using an anti-human FSH serum. In extracts from infected wild type *Nicotiana tabacum* L. cv. Wisconsin plants, a prominent band migrating at about 30 kDa was observed, representing the sc-bFSH translation product. In addition, bFSH derivatives with higher electrophoretic mobility were observed on the blot, suggesting proteolytic breakdown of the initial translation product. In extracts from infected StayGreen *Nicotiana tabacum* L. cv. Wisconsin plants more intact sc-bFSH had accumulated and significantly less degradation products were observed. These results demonstrate that also virus encoded recombinant proteins can be more efficiently and to a higher homogeneity be produced in StayGreen background.

### Improved systemic infection of recombinant viruses in StayGreen plants

*Nicotiana tabacum* L. cv. Wisconsin and *Nicotiana tabacum* L. cv. Wisconsin StayGreen plants (the latter carrying the pSAG12-*ipt* constructs) were infected with recombinant PVX viruses carrying the gene encoding the Green Fluorescent Protein essentially as described by Turpen et al. (1998). Subsequently, progress of the infection and expression of the GFP protein was studied. It was evident from the experiments that, as compared to the wild type plants, infection of the StayGreen plants was more efficient and that the recombinant viruses were spread over larger areas of the StayGreen plants. These results show that from virus infected StayGreen plants, not only more recombinant protein can be recovered per gram fresh weight, but that also more biomass containing the desired product can be harvested.

### References to example 3

1. Sugahara,T., Sato,A., Kudo, M., Ben Menahem D., Pixley MR., Hsueh AJ. and Boime, I. (1996) Expression of biologically active fusion genes encoding the common alpha subunit and the follicle-stimulating hormone beta subunit. Role of a linker sequence. J.Biol.Chem, 271(18) : 10445-8.
2. Kumagai M.H., Turpen T.H., Weinzettl N.,Della-Cioppa G., Turpen A.M., Donson J., Hilf M.E., Grantham G.L., Dawson W.O., Chow T.P., Piatak M. and Grill L.K. (1993) Rapid high-level expression of biologically active alpha-tricosanthin in transfected plants by an RNA viral vector. Proc. Natl Acad. Sci. USA 90, 427-430.
3. Turpen, T.H. and Reinl, S.J. (1998) Tobamovirus vectors for expression of recombinant genes in plants. In Methods in Biotechnology, vol 3, Recombinant Proteins From Plants: Production and Isolation of Clinically Useful Compounds. Ed. C. Cunningham and A.J.R. Porter. Donson J., Kearney C.M., Hilf M.E. and Dawson W.O. (1991) Systemic expression of a bacterial gene by tobacco mosaic virus-based vector. Proc. Natl Acad. Sci. USA 88, 7204-7208.

### Figure legends

**Figure 1.** Coomassie-stained 12% SDS-PAGE gel (reducing conditions) showing the proteins from the subsequent fractions obtained during the antibody purification procedure. Lane 1: crude leaf extract; lane 2: 20-60% ammoniumsulphate fraction; lane 3: fraction retained on Protein G column; lane 4: Cation-exchange peak I; lane 5: Cation-exchange peak II.

**Figure 2.** Coomassie-stained 10% SDS-PAGE gel showing (A) mouse hybridoma MGR48 antibody, (B) purified total plantibody (fragments), (C) cation-exchange fraction I of total plantibody (fragments), and (D) cation-exchange fraction II of total plantibody (fragments), run under non-reducing and reducing conditions (i.e. without and with β-mercaptoethanol, respectively). M: molecular weight marker proteins.

**Figure 3.** Number of leaves and stem length of the transgenic plants grown under four different climate conditions, i.e. at 15 °C/high irradiation, 15 °C/low irradiation, 25 °C/high irradiation, and 25 °C/low irradiation.

**Figure 4.** Production of dry weight biomass as lateral shoots, stems and leaves by the transgenic plants grown at 15 °C/high irradiation, 15 °C/low irradiation, 25 °C/high irradiation, and 25 °C/low irradiation. The stacked columns represent the avarage results of three experiments; bars show SD.

**Figure 5.** Total soluble protein content in top, middle and base leaves of the transgenic tobacco plants grown at 15 °C/high irradiation, 15 °C/low irradiation, 25 °C/high irradiation, and 25 °C/low irradiation. Bars show SD (n=3).

**Figure 6.** IgG content in top, middle and base leaves of the transgenic tobacco plants grown at 15 °C/high irradiation, 15 °C/low irradiation, 25 °C/high irradiation, and 25 °C/low irradiation. Bars show SD (n=3).

**Figure 7.** Ratio between relative amount of H chain and relative amount of protein that exhibits L chain mobility (i.e. L chain and putative H chain fragment; L') in top, middle and base leaves of the transgenic tobacco plants grown at 15 °C/high irradiation, 15 °C/low irradiation, 25 °C/high irradiation, and 25 °C/low irradiation. Bars show SD (n=3).

**Figure 8.** Immunoblots showing the H chain (H) and the protein band that exhibits L chain mobility (i.e. L chain and putative H chain fragment; L') of the MGR48 plantibody in the course of its incubation with crude protein extracts of top, middle and base leaves from wild-type tobacco. The incubations were performed at pH 4.5 with 25 *µ*g.ml⁻¹ of purified plantibody and 58 *µ*g.ml⁻¹ of leaf protein. Similar results were obtained in two independent experiments.

**Figure 9.** Proteolytic degradation of the MGR48 plantibody in the course of separate incubations with crude extracts of top (□), middle (●) and base (O) leaves from wild-type tobacco. The incubations were performed at pH 4.5 with 25 *µ*g.ml⁻¹ of purified plantibody and equal volumes of leaf extract, the latter corresponding with equal amounts of fresh weight. The amount of H chain is expressed as percentage of the initial amount and was quantified by densitometry of the H bands on immunoblots.

**Figure 10.** *In vitro* proteolytic degradation of MGR48 antibody from tobacco (●) and of MGR48 antibody from mouse hybridoma cells (O) at pH 4.5. The parallel incubations were performed with an equal amount of antibody (31 *µ*g.ml⁻¹) and an equal volume of the same crude leaf extract from wild-type tobacco. The amount of H chain is expressed as percentage of the initial amount and was quantified by densitometry of the H bands on immunoblots. Similar results were obtained in three independent experiments.

**Figure 11.** Amount of IgG expressed per amount of total protein in top, middle and base leaves of the transgenic tobacco plants grown at 15 °C/high irradiation, 15 °C/low irradiation, 25 °C/high irradiation, and 25 °C/low irradiation. Bars show SD (n=3).

**Figure 12.** Immunoblot of total soluble protein extracts from top (A), middle (B) and two base (C and D) leaves of MGR48 expressing tobacco plants that were crossed with tobacco plants carrying the pSAG12-*ipt* gene (left), and wild-type tobacco (right). The lanes contained equal volumes of protein extract, corresponding with equal amount of leaf tissue. Mw: molecular weight.

**Figure 13.** Mass 93 chromatograms of leaves of lis-transformed Petunia hybrida plants. Upper trace: youngest leaf, middle trace: older leaf, lower trace: oldest leaf.

## Claims

1. A method to increase the level of a desired product obtainable from a plant provided with a recombinant nucleic acid comprising allowing said plant to synthesise said product further comprising providing for prevention of degradation of said product.

2. A method according to claim 1 wherein said degradation is prevented by at least partly preventing catabolisation of said product.

3. A method according to claim 1 or 2 wherein said degradation is prevented by delaying senescence of said plant.

4. A method according to claim 3 wherein said senescence is delayed by the gene product of a nucleic acid with which said plant or an ancestor of said plant has been provided.

5. A method according to claim 4 wherein said gene product comprises protein.

6. A method according to claim 5 wherein said protein comprises a regulator or catalyst, or functional fragment thereof, of the biosynthesis of a plant growth regulator such as a plant hormone.

7. A method according to anyone of claims 3 to 6 wherein said senescence comprises leaf senescence.

8. A method according to anyone of claims 1 to 7 wherein said plant is provided with a recombinant nucleic acid comprising a viral vector.

9. A recombinant plant capable of producing a desired product comprising a gene product allowing at least partly preventing degradation of said desired product.

10. A plant according to claim 9 wherein said desired product comprises a proteinaceous substance.

11. A plant according to claim 10 wherein said proteinaceous substance comprises an antibody or functional fragment or functional equivalent thereof.

12. A plant according to claim 9 wherein said desired product comprises a primary or secondary metabolite.

13. A plant according to anyone of claims 9 to 12 wherein said gene product comprises protein.

14. A plant according to claim 13 wherein said protein comprises a regulator or catalyst, or functional fragment thereof, of the biosynthesis of a plant growth regulator such as a plant hormone.

15. A method for obtaining a desired plant product comprising cultivating a plant according to anyone of claims 9 to 14 to a harvestable stage and harvesting said plant or parts thereof.

16. A method according to claim 15 further comprising extracting said desired product from said plant.

17. A plant product obtainable by a method according to claims 15 or 16.
